(19) Europäisches Patentamt / European Patent Office / Office européen des brevets

(11) **EP 3 538 050 B1**

(12) **EUROPEAN PATENT SPECIFICATION**

(45) Date of publication and mention
of the grant of the patent:
**18.10.2023 Bulletin 2023/42**

(21) Application number: **17868920.4**

(22) Date of filing: **14.11.2017**

(51) International Patent Classification (IPC):
**A61H 23/02** (2006.01)        **A61H 1/00** (2006.01)
**A61B 5/00** (2006.01)

(52) Cooperative Patent Classification (CPC):
**A61H 23/0218; A61B 5/4023;** A61H 2201/0149;
A61H 2201/1604; A61H 2201/165;
A61H 2201/5005; A61H 2201/5084; A61H 2205/02;
A61H 2230/06; A61H 2230/50; A61H 2230/65

(86) International application number:
**PCT/US2017/061520**

(87) International publication number:
**WO 2018/089994 (17.05.2018 Gazette 2018/20)**

(54) **DEVICES AND METHODS FOR REDUCING THE SYMPTOMS OF MALADIES OF THE VESTIBULAR SYSTEM**

VORRICHTUNGEN UND VERFAHREN ZUR VERRINGERUNG DER SYMPTOME VON LEIDEN DES VESTIBULÄREN SYSTEMS

DISPOSITIFS ET PROCÉDÉS POUR RÉDUIRE LES SYMPTÔMES DE MALADIES DU SYSTÈME VESTIBULAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.11.2016   US 201662421708 P
07.04.2017   US 201715481457**

(43) Date of publication of application:
**18.09.2019 Bulletin 2019/38**

(73) Proprietor: **Otolith Sound, Inc.
Washington DC 20009 (US)**

(72) Inventors:
• **OWEN, Samuel
Alexandria, Virginia 22303 (US)**
• **TRUE, Robert
Washington DC 20009 (US)**

(74) Representative: **Cooley (UK) LLP
22 Bishopsgate
London EC2N 4BQ (GB)**

(56) References cited:
EP-A1- 3 054 702          WO-A1-2009/029040
WO-A1-2015/143053        WO-A2-00/10361
US-A- 6 077 237          US-A1- 2011 071 340
US-A1- 2016 089 298      US-A1- 2016 256 347
US-B1- 6 210 321

**Description**

**BACKGROUND**

Orientation, balance, position, and movement of a body can be determined by the brain through a combination of signals received from various parts of anatomy, including eyes, ears, and muscles. For example, movement of endolymph fluid in the vestibular system of the inner ear can be sensed by nerve cells with hair follicles to determine movement and orientation of the head, otoliths in the vestibular system of the inner ear sink in the direction of gravity and pull on hair follicles of nerve cells to aid in distinguishing up from down; horizontal and vertical visual patterns received by the eyes can assist with determinations of orientation, balance, and position; and differential strain on opposing neck muscles can help determine head position and orientation. When signals from these sources do not match, an individual can develop motion sickness, experience vertigo, or even become unconscious. Unmatched orientation, balance, position and movement signals can be the result of extreme or unfamiliar movement during, for example, travel in cars, trains, airplanes, and other modes of transportation, or can be the result of simulated perceived movement during, for example, 3D movies, 3D video games, and virtual reality devices.

In a natural adaptive response, a brain can ignore sensory information in signals that are chaotic, not novel, or unintelligible. For example, it is believed that repetitive vibrations in the vestibular system of the inner ear decreases the amplitude of electrical signals sent to the cerebellum (see, for example, H. Sohmer et al. Effect of noise on the vestibular system - Vestibular evoked potential studies in rats. 2 Noise Health 41 (1999)).

Vibrations can be very therapeutic. Accordingly, there exists massage devices used on several parts of the body to produce soothing sensations from vibrations. One technology that is used to create these vibrations is a surface or bone conduction transducer. However, when such devices are used on the head the noise they create can be irritating when used close to a person's ear. This occurs in large part because the resonant frequencies of the vibrations generated by such a transducer cause audible and irritating tones. Additionally, existing bone conduction transducers are inefficient when used to create powerful vibrations because they produce a large spectrum of frequencies when only a small narrow band may be needed.

Accordingly, the present invention provides novel and non-obvious systems that improve the treatment of motion sickness as well as other maladies that are associated with the vestibular system of an individual.

Additionally, the systems provided by the invention utilize novel and non-obvious vestibular system affecting/agitating/stimulating devices (e.g., transducers) that overcome the limitations of existing devices.

US2016/0089298 discloses a device for mitigating motion sickness and other responses to inconsistent sensory information. The device has a signal generator that is connected to a battery and generates a signal that contains a tone with a sine wave pattern. The signal contains an average output power level of between 100 and 150 decibels. A vibration-inducing element is connected to the signal generator and converts signal into physical vibrations. The battery, signal generator and vibration-inducing element are placed in a portable frame against the skull.

**BRIEF DESCRIPTION OF THE DRAWINGS**

Figure 1 depicts a simplified block diagram of an exemplary system according to an embodiment of the invention.
Figure 2 depicts a cut-away view of an exemplary device according to an embodiment of the invention.
Figure 3 depicts an alternative view of an exemplary device according to an embodiment of the invention.
Figure 4 depicts an exemplary system combined with audio headphones according to an embodiment of the invention.
Figures 5 and 6 depict another exemplary system according to embodiment of the invention.
Figure 7 depicts yet another exemplary system according to another embodiment of the invention.

**SUMMARY**

The inventors recognized that a "noisy" signal from the vestibular system cannot be properly interpreted by the brain, prompting the brain to instead rely on signals from other sources, such as the eyes and muscles. Fewer signals to interpret allows the brain to determine orientation, balance, position, movement, or a combination thereof, with a smaller chance of unmatched signals, consequently reducing the likelihood that an individual may experience resulting detrimental physiological effects, such as motion sickness.

Accordingly, the present invention makes use of this discovery to generate signals that de-stimulate the vestibular system of an individual. The present invention provides exemplary devices that generate such signals and apply them, for example, to the head (i.e., skull) of an individual so that the signals may travel to the vestibular system of an individual. When received by the vestibular system the signals effectively de-stimulate the vestibular system by, for example, masking other signals that stimulate the vestibular system.

The systems and devices described herein may be combined or connected to detection circuitry for detecting nystagmus (i.e., shaky eye). Such a measurement may be used to measure how well the vibratory signals provided by systems and devices of the present invention de-stimulate the vestibular system by masking stimulating signals. Alternatively, instead of detecting a nystagmus, the detection circuitry may detect signals from the vestibulochochlear nerve itself.

One exemplary embodiment is a system for reducing the symptoms of maladies of the vestibular system, where the system comprises a vestibular device operable to generate one or more vibratory signals at power levels that cause a de-stimulation of the vestibular system of an individual, wherein the device comprises (i) a signal generator for generating one or more initial signals; (ii) an amplifying section for receiving the one or more initial signals (e.g., a sine wave) and amplifying the signals by an amount sufficient to produce one or more signals that de-stimulate the vestibular system; and (iii) a vibration generating element (e.g., a transducer) operable to receive the one or more amplified signals and generate one or more fluctuating, vibration signals.

An exemplary transducer may be operable to generate the one or more vibration signals, wherein a majority of the power in each of the vibratory signals is contained in a fundamental frequency of each of the vibration signals.

In addition, the exemplary transducer may include, among other elements, a coil operable to generate a magnetic field and apply the field to a type of magnet, wherein the magnet is operable to oscillate at a resonant frequency to generate the one or more vibration signals.

The systems and devices provided by the present invention may be customized for a given individual (e.g., patient) at a particular power and frequency of operation. Accordingly, an inventive device may include a power supply (e.g., battery), signal generator, amplifier, and battery charger.

In accordance with one aspect of the invention, there is provided a system for reducing the symptoms of maladies of the vestibular system as defined by claim 1. Optional features are defined by the dependent claims.

## DETAILED DESCRIPTION, INCLUDING EXAMPLES

Exemplary embodiments of systems, devices and methods for reducing the symptoms of maladies related to the vestibular system, such as motion sickness, are described herein. Although specific exemplary embodiments are discussed herein, there is no intent to limit the scope of the present invention to such embodiments. To the contrary, the exemplary embodiments discussed herein are for illustrative purposes. Modified and alternative embodiments may be implemented without departing from the scope of the present invention. Said another way, the exemplary embodiments presented herein are only some of the many that fall within the scope of the present invention, it being practically impossible for the inventor to describe all the many possible exemplary embodiments and variations that fall within the scope of the present invention.

For example, though the systems, devices and methods described herein focus on applying the inventive systems and methods close to the ear of an individual, this is for illustrative purposes only, it being further understood that the systems, devices and methods may be applied to many different parts of an individual's head.

It should also be understood that one or more exemplary embodiments may be described as a process or method. Although a process/method may be described as sequential, such a process/method may be performed in parallel, concurrently or simultaneously. In addition, the order of each step within a process/method may be rearranged. A process/method may be terminated when completed, and may also include additional steps not included in a description of the process/method.

As used herein, the term "and/or" includes all combinations of one or more of the associated listed items. As used herein, the singular forms "a," "an" and "the" are intended to include the plural form, unless the context and/or common sense indicates otherwise. It should be further understood that the terms "comprises", "comprising,", "includes" and/or "including", when used herein, specify the presence of stated features, systems, subsystems, steps, operations, elements, and/or components, but do not preclude the presence or addition of one or more other features, systems, subsystems, steps, operations, elements, components, and/or combinations thereof.

When used herein the phrases "connection", "connected to", or similar phrases means an indirect or direct physical connection between at least two different parts of a system or device or means one part of a system or device subsumed within (and thereby connected to) at least one other part of a device. When one part of a system or device is described or depicted as being connected to another part, other well-known components used to facilitate such a connection may not be described or depicted because such components are well known to those skilled in the art.

Yet further, when one part of a system or device is described or depicted as being connected to another part using "a connection" (or single line in a figure) it should be understood that practically speaking such a connection (line) may comprise (and many times will comprise) more than one physical connection or channel, may be omnidirectional or bi-directional, may or may not include separate data, formatting and signaling.

It should be noted that the systems and devices illustrated in the figures are not drawn to scale, are not representative of an actual shape or size and are not representative of any actual device layout, manufacture's drawing or visual. Rather, the systems and devices are drawn to simply help explain the features, functions and processes of exemplary embodiments of the present invention described herein.

As used herein, the term "embodiment" refers to one example of the present invention.

The present invention provides for systems, devices and related methods which generates one or more de-stimulating signals sufficient to affect the vestibular function of an individual. This altered vestibular function may be perceived through quantitative measurements such as induced nystagmus and changes in vestibular evoked potentials or by qualitative measurements such as relief from motion sickness, virtual reality sickness, vertigo, or any other vestibular condition.

One embodiment of the invention to mitigate motion sickness is by disrupting, controlling, or influencing anatomy of the vestibular system, such as, for example, otoliths, endolymph, and hair follicles. An embodiment of the invention may induce vibrations in the vestibular system, including otoliths and/or semicircular canals, of the inner ear, thereby masking the sensory information sent to the brain from the vestibular system. Because of the constant and non-informative nature of this masked sensory information, the brain, as part of a normal physiological response, may rely less on signals received from the vestibular system and rely more heavily on other sources, thereby mitigating motion sickness. The vibrations created may be constant or vary over a 1/3 octave range to ensure the body doesn't adapt to the masking. Another embodiment of the invention may induce vibrations in the vestibular system of the inner ear, thereby controlling the positions of otoliths, endolymph, hair follicles or combinations thereof, and, consequently, alter the sensory information in the signal sent from the vestibular system to the brain to mitigate motion sickness.

In another embodiment, the signals generated by the systems and device may be de-stimulating, vibratory signals that are transmitted to the brain.

The systems and devices described above and elsewhere herein may be combined or connected to detection circuitry for detecting nystagmus (i.e., shaky eye). Such a measurement may be used to measure how well the vibratory signals provided by the present invention masks the vestibular system. Alternatively, instead of detecting a nystagmus, the detection circuitry may detect signals from vestibular nerve itself.

The systems and devices provided by the present invention may be customized for a given individual (e.g., patient) at a particular power and frequency of operation. Accordingly, an inventive system or device may include a power supply (e.g., battery), signal generator, amplifier, and battery charger.

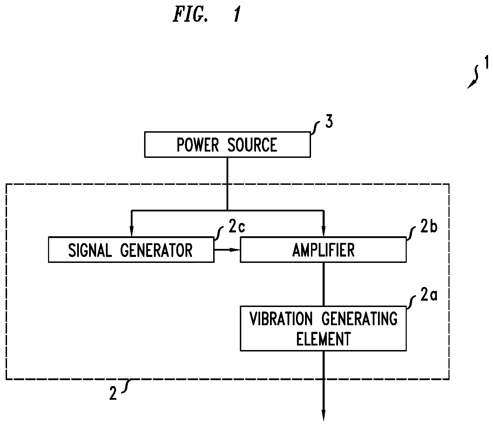

Referring now to Figure 1, there is depicted an exemplary system 1 for de-stimulating the vestibular system. As depicted the system 1 comprises a vestibular device 2 operable to generate one or more vibratory signals (i.e., vibrations) at power levels that cause a de-stimulation of the vestibular system of an individual when the system 1 is placed on, or near, the vestibular system. In particular, the vestibular device 2 of the system 1 may be positioned on an individual's head where there is little tissue allowing the signal to propagate thru to the vestibular system (e.g., over the mastoid bone).

In one embodiment, the vestibular device 2 may comprise a vibration generating element 2a (e.g., a transducer) that is operable to produce one or more fluctuating, vibration signals at one or more harmonics of a frequency in the range 10 to 300 Hz, for example. The inventors believe that such signals de-stimulate the vestibular system, or alternatively may cause the system to be confused, by masking other signals that may stimulate the vestibular system. The one or more signals may take the form of a sine wave or a number of other signal types.

The present inventors conducted a number of experiments with experimental devices. The evidence obtained by the inventors indicates that when the system 1 generates vibratory signals (vibrations) at low frequencies it is important to ensure that the waveform (e.g., sine wave) of such a signal remains unchanged when pressure (force) is applied to the system 1, and in particular device 2, as the device 2 is pressed to the skull of an individual.

In more detail, the device 2 may further comprise a signal generator 2c for generating one or more initial signals (e.g. 50 to 67 Hz.) such as a pure sine wave and an amplifying section 2b for receiving the one or more signals generated by the generator 2a and amplifying the one or more signals by an amount (e.g. 0.5 watt to 3 watts) sufficient to produce one or more signals that de-stimulates the vestibular system. The so amplified signal is then provided to the vibration generating element 2a that, upon receiving the amplified signal generates the one or more vibratory signals.

In one experiment, the inventors conducted an experiment using a system similar to system 1 where a pure sine wave signal was generated by the combination of generator 2c and amplifier 2b and then sent to the vibration generating element 2a (e.g., a transducer). Thereafter, no pressure was exerted. The result was that a pure sine wave was detected on a testing device. However, when pressure was applied, the pure sine wave signal that was generated changed its characteristics because much of the energy derived from the amplified electrical signal was physically converted from the signal's fundamental frequency to its higher harmonics. During further testing the inventors discovered that transducers which remained the most fundamental (i.e., where the majority of the power is contained in a fundamental frequency, and not in harmonics) were the most effective at de-stimulating the vestibular system in order to reduce the symptoms of motion sickness for individuals riding in cars and virtual reality sickness for those using virtual reality devices. Accordingly, the inventors concluded that transducers that are operable to generate one or more vibration signals, wherein a majority of the power in each of the vibratory signals is contained in a fundamental frequency of each of the vibration signals are highly desirable. Further, that transducers that are utilized within systems provided by the present invention should be operable to generate a signal of pure tone regardless of the power of the signal (i.e., a sine wave at a given signal).

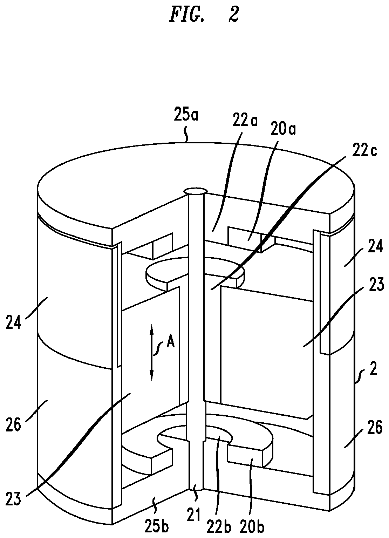

Referring now to Figure 2 there is depicted an exemplary vestibular device 2 (e.g., a transducer) according to one embodiment. As depicted, the device 2 may comprise a first type of magnet 20a,20b, a second type of magnet 23, a coil 24, a bushing 22c and pin 21. As power is applied to the device 2 (through means and connections known in the art, and omitted for the sake of clarity in Figure 2) using an electrical signal that comprises a sine wave or another type of signal structure at a desired frequency (e.g., 10 Hz to 300 Hz), the coil 24 is operable to generate a magnetic field with an induced electrical current. The magnetic field in turn will apply a magnetic force on magnet 23. The fields, when applied to the magnet 23, causes the magnet 23 to move as indicated by the labelled arrow "A" in Figure 2. Substantially simultaneously, magnets 20a and 20b each create a constant magnetic field, each of which is applied to magnet 23 (i.e. North side of magnet 20a will face the North side of magnet 23 and the South side of magnet 20b will face the South side of magnet 23). These applied, opposing forces created by magnets 20a, b are operable to control the magnet 23 such that the magnet 23 oscillates around an equilibrium position where it naturally vibrates at a resonant frequency of (spring constant / mass of magnet 23, ^0.5 where the spring constant is defined as the restorative force divided by the distance magnet 23 has moved from its equilibrium position) and generates the one or more vibration signals. In one embodiment, the induced electrical signal matches the resonant frequency magnet 23 vibrates at due to magnets 20a and 20b, (i.e., it will cause the magnet 23 to oscillate or move back and forth, or up and down depending on your perspective) along the axis indicated by the labelled arrow "A". This axis may substantially correspond with the axis of the pin 21.

In an embodiment of the invention to ensure that the magnets 20a, b and 23 do not oscillate or wobble in an undesired fashion (along an axis other than that indicated by the labelled arrows "A"), which would affect the efficiency of the system and increase undesirable friction that causes secondary vibrations (i.e., a humming sound), the magnet 23 is configured such that its motion is restricted by the rod/pin 21. In one embodiment, each of the magnets 20a, b is secured to the end caps 25a, b of the transducer 2 with a glue, epoxy, or another form of adhesive, magnet 23 is fitted with a bushing 22c which smoothly moves over rod/pin 21 while restricting any motion that's not parallel to "A", and a glue, epoxy, or another form of adhesive may also be used to secure the rod/pin 21 to the end caps 25a, b through a fitted hole 22 a, b.

It should be understood that the magnets 20a, b are one example of elastic objects. In an alternative design, the magnets 20a, b may be replaced by other elastic objects which create a narrow resonance (defined in part by their spring constant). For example, the elastic objects may consist of springs of any form and material, where the springs may be supported by (i) cavities (not shown in Figure 2) in end caps 25a, b. (ii) pins extruding from the end caps (not shown in Figure 2), (iii) silicon foam or foam of any other material (not shown in Figure 2), or (iv) are pieces of rubber or any another solid elastic material (not shown in Figure 2). When springs are used as the elastic objects a glue, epoxy, or any form of adhesive may be used to secure the springs to other parts of the device 2 (e.g., to the magnet, tubing, and/or end caps 25a, b).

In embodiments of the invention, the springs are operable to reduce the chances for the magnet 23 to make contact with the inner diameter of the tubing 26 along an axis other than the axis represented by the labelled arrow "A", In embodiments, the rod/pin 21 and bushing 22c may be included for support or be excluded depending on the springs utilized (i.e., depending on whether the springs sufficiently restrict the magnet 23 motion).

In embodiments of the invention, the tubing 26 may contain a lubricant, such as ferrofluid, Teflon, or another lubricant that reduces the friction that may occur when the magnet 23 may meet the inner diameter of the tubing 26. The less friction the "quieter" the device 2 (i.e., the less noise generated by such contact). Such lubricants may also be used to reduce the friction between the bushing and pin.

The present invention includes embodiments where the outer surface of the tubing 26 and/or endcaps 25a, b are covered with a sound absorbing material. Further, in an embodiment one or more of the endcaps 25a, b may be covered with a friction reducing material, such as cork for example, so that when an endcap or endcaps 25a, b come in contact with a person's skin or body the contact is less abrasive than if the endcaps 25a, b where not covered by such material. Further, in an embodiment one or more of the endcaps 25a, b may be covered with a padded material, such as cork for example, so that when an endcap or endcaps 25a, b come in contact with a person's skin or body the contact is spread over a larger area reducing the pressure if the endcaps 25a, b where not covered by such material.

As described above, either magnets or springs may be used as the elastic objects. It should be understood, however, that magnets and springs are only two of the many types of elastic objects that may be used.

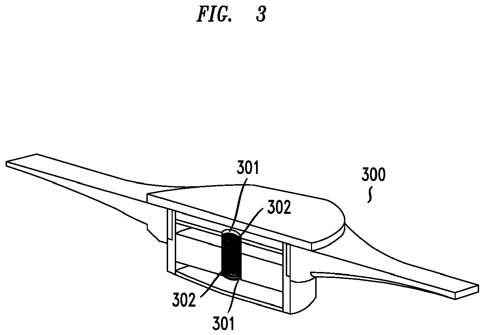

Figure 3 depicts another view of a vestibular device 300 that utilizes springs as the elastic objects according to an embodiment of the present invention.

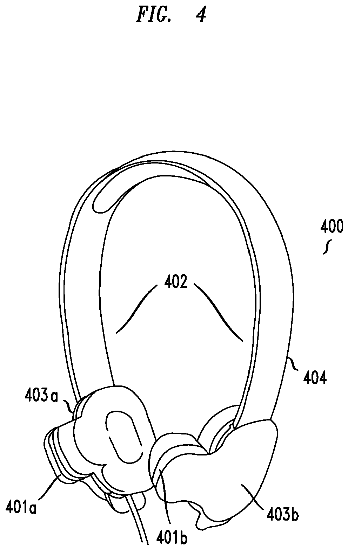

Referring now to Figure 4 a system 400 for reducing the effects of maladies related to the vestibular system (e.g., motion sickness) is shown. As shown the system 400 may comprise two vestibular devices 40 1a, b (similar to device 2 or 300) that may be integrated into, or connected to a first type of audio headphones 402 (e.g., headphones without noise cancellation circuitry). In such an embodiment, a user (not shown in Figure 4) has the option to just listen to music, listen to music and manipulate their vestibular, or just manipulate their vestibular system. In another embodiment, the headphones 402 may comprise audio headphones that include noise cancellation circuitry. The headphones 402 may be integrated with, or connected to, the vestibular devices 401a, b so the user has the option to listen to music, listen to music and manipulate their vestibular, listen to music and cancel noise, manipulate their vestibular system and cancel noise, listen to music manipulate their vestibular system and cancel noise, just manipulate their vestibular system or just cancel noise.

The embodiment depicted in Figure 4 illustrates the use of more than one vestibular device. It should be understood that each of the systems described herein may include more than one vestibular device (e.g., at least two devices).

The noise cancellation circuitry in the system 400 may also be used to reduce the level of audible sound caused by vibrations produced by the vestibular device 401 (e.g., a vestibular device described herein) while allowing substantially all other sounds to be received by a person's vestibular system. In more detail, because the physics of vibrational signals are predictable, the system 400 may include noise cancellation circuitry that generates a signal (or signals) that is similar to the audible signals produced by the vestibular device 401, but is out of phase with such a signal (e.g., 180 degrees). Such an out of phase signal acts to reduce the signal level of such audible signals detected by a person's vestibular system so that a person may not hear the audible sounds.

When used in conjunction with headphones 402 the vestibular devices 401a, b may be placed in a plurality of positions. One such position for each device 401a, b is located a distance away from a respective audio speaker 403a, b that are a part of the headphones 402, along the elongated portion 404 (e.g., band) of the headphones 402. This would allow the elements of the vestibular devices 401a, b, that are responsible for generating vibrational signals, to be a placed at positions aside, or on top of: a person's head at positions that have been shown to be effective for delivering the vibrational signals to the vestibular system (e.g., positioned on the mastoid, zygomatic arch, parietal bone, etc.). Placement along the elongated portion 404 also takes advantage of the ergonomics of the elongated portion 404 (i.e., the shape of the human head is more consistent than the human ear).

Alternatively, the vestibular devices 401a, b may be incorporated earcups of headphones 402that may be co-located with the speakers 403a, b so that the ornamental shape or profile of the headphones 402 are not affected, Alternatively, the vestibular device 401 may be incorporated into an attachment which attaches and detaches to a pair of headphones so that the user may choose to have just the headphones or have the headphones with the vestibular device.

Yet further, in another embodiment a vestibular device described elsewhere hereon may be integrated into, or connected to a seat (car seat, office chair, etc.) and configured so that, for example, when a user's head rests against the seat the advantageous effects of the vestibular device are maximized. Alternatively, the vestibular device may be removably attached to the seat such that it can be removed and placed on the head of a user. Still further, a vestibular device may be integrated into, or connected to, a physical platform (e.g., a pad) that may be mounted in a vehicle at a position where an individual may rest their head on, or near, the vestibular device. Exemplary positions may be the seat of a vehicle, the window of a vehicle or a horizontal surface of a vehicle's rear seat.

Still further, a vestibular device as described herein may be integrated into, or connected to, a pillow, travel pillow, or cushion (collectively "pillow"), In such an embodiment, the combination of pillow and vestibular device may be configured such that when a user rests their head on the vestibular device, and receives vibrations generated by the device, their head is still supported.

There are many more systems that may include a vestibular device described herein. One of these is a system that includes a vestibular device and a virtual reality device (e.g., headset). In one exemplary design, a mounting lattice can be configured to secure the vestibular device against a person's head so that the effects of the vestibular device (i.e., its vibrations) can be effective, or the vestibular device may be connected to the virtual reality headset such that it can be removed and placed on a person's head.

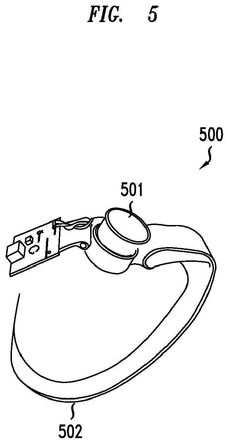

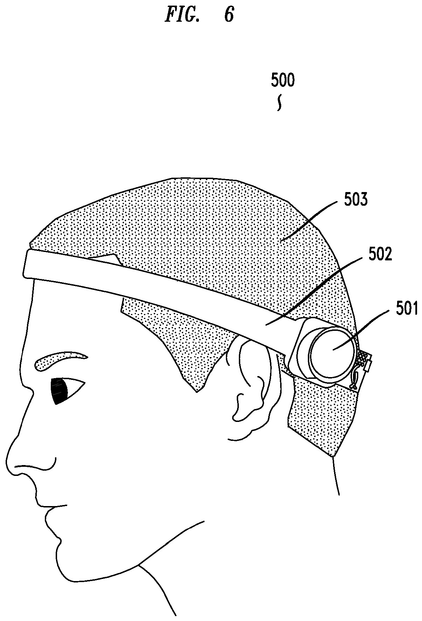

Figures 5 and 6 depict additional embodiments of the invention. As depicted, a system 500 may include a vestibular device 501 described herein integrated into, or connected to, a headband 502. The headband 502 may comprise an elastic, Velcro, metal or plastic, or another material that permits the headband 502 to hold the vestibular device 501 (i.e., the vibration generating element of the device 501) anywhere on the head 503 of an individual in order to allow the signals for de-stimulating or masking the vestibular system of the individual The system 500 may be equipped with a power source (e.g., a battery) to power it (not shown in Figures 5 or 6) or it may be attached via a wire to another power source (e.g., AC or DC source, such as a battery pack; not shown in figures) which is not a part of the headband 502. The system 500 may include the necessary electrical driving circuitry to drive the vestibular device 501. Alternatively, such circuitry and power source may be connected to the vestibular device 501 through means known in the art (e.g., electrical wires, optical fiber).

In a further embodiment, a system may include a vestibular device described herein that is integrated into, or connected to, glasses or sunglasses. In an embodiment, the vestibular device may be attached to a part of glasses that make contact and rest on a person's ear (e.g., temple tips). As before, such a system may include a power source (e.g., battery) and signal driving circuitry or such circuitry and power source may be connected to the vestibular device by means known in the art.

In a yet a further embodiment, a system may include a vestibular device described herein that is integrated into, or connected to, a hat or cap (collectively "hat"). The system may include a power source (e.g., battery) and electrical driving circuitry or such circuitry and power source may be connected to the system by means known in the art.

In still a further embodiment, a system may include a vestibular device described herein that may be integrated into, or connected to a hair accessory (an object that holds on to the hair for stability). The accessory may be ornamental or not. By attaching the system to the hair of a person the vestibular device may make contact with the head of a person. The system may include a power source (e.g., battery) and electrical driving circuitry or such circuitry and power source may be connected to the system by means known in the art.

In a further embodiment, a system may comprise a vestibular device described herein integrated into, or connected to (a) a soldiers' helmet or any other form of helmet, or (b) a pilots' headset or any other type of headset. As before, the system may include the necessary power source (e.g., battery) and electrical driving circuitry. Such a system may de-stimulate the vestibular system of a pilot, thus allowing the pilot to rely on an airborne vehicle's (e.g., a plane) instruments for orientation.

Alternative embodiments of the present invention provide for systems that include a vestibular device described herein integrated into, or connected to, sensing means or sensor (collectively referred to as "sensor") (e.g., body sensor, environmental sensor, a temperature sensor, acceleration sensor, skin conduction sensor, heart rate monitor, a glucose sensor), electrodes which stimulate the vestibulocochlear nerve or a caloric stimulator such as the one described in U.S. Patent Application No. 20140309718.

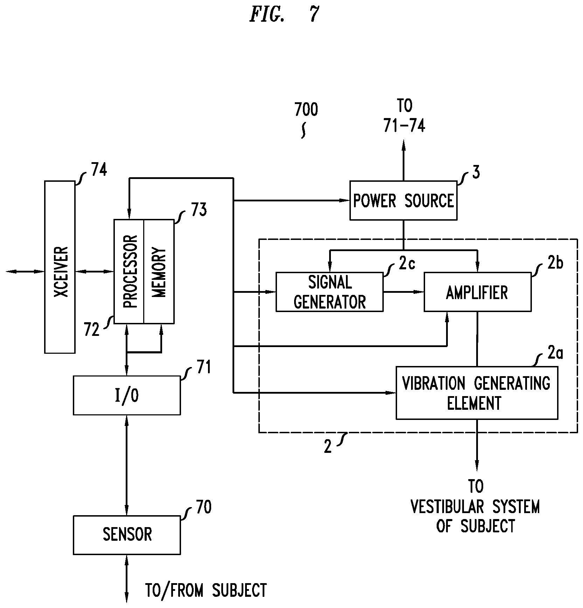

For example, Figure 7 depicts a system 700 that includes a vestibular device 2 similar to the device in Figure I, for example. In addition, the system 700 may include a sensor 70, input/output (I/O) section 71, processor 72, memory 73, and transceiver 74. In an embodiment, data from sensor 70 may be sent to the processor 72 and/or stored in memory 73 via I/O section 71. The data may consist of analog or digital signals output from the sensing means representative of real-world measurements of factors relevant to detecting or treating symptoms of maladies of the vestibular system. For example, sensors may detect temperatures, noise levels, vibrations, accelerations/de-accelerations, velocities, pressures, moisture etc., and input this information into memory 73 or the processor 72. Thereafter, the processor 72 may be operable to execute instructions stored in its memory (not shown) or in memory 73 to control the operation of other elements depicted in Figure 7. For example, the processor 72 may adjust the operation of the signal generator 2c (e.g., adjust the output frequency, structure of the signal), amplifier 2b (e.g., increase or decrease amplification) and vibrational generating element 2a. Yet further, the data may be stored for historical purposes in memory 73 and analyzed by processor 72 using additional instructions stored in its memory or memory 73 order to determine the proper settings of system 700 to customize the operation of system 700 for a particular individual and, thereby, customize the treatment of symptoms associated with maladies of the vestibular system. Still further, the data may be sent to a remote computing device via transceiver 74 for storage or further analysis. Conversely, data and signals may be received from such a remote computing device (not shown in Figure 7) via transceiver 74, and then used by the processor 72 to control (e.g., adjust) the operation of elements of the system 700 to customize the treatment of symptoms associated with maladies of the vestibular system. In an embodiment, the processor 72 may access instructions stored in its own memory or in memory 73 to make such adjustments.

Additional embodiments include a system that includes a vestibular device described herein that is integrated into, or connected to a simulator, virtual reality device, augmented reality device, playground device, or amusement park ride device (e.g., the seat of an amusement park roller coaster, twisting ride, or ferris wheel, for example).

In addition to relieving the symptoms associated with motion sickness, the systems provided by the present invention may be effective at relieving the symptoms associated with vertigo, Meniere's disease, labyrinthitis, vestibular migraine, Benign Paraxial Positional Vertigo (BPPV), damage to the vestibular system, 'the spins' from alcohol or other drug consumption, tinnitus or any other vestibular or balance disorder.

Further, the systems provided by the present invention described herein may be used to diagnose whether dizziness is brought on by a vestibular dysfunction or something else, such as a stroke.

Further, the systems provided by the present invention described herein may be used to relieve, distract, or mask the symptoms of tinnitus. The inventors have completed several tests with experimental devices that illustrate the effectiveness of using de-stimulating or masking signals to treat symptoms of maladies of the vestibular system. The following summarily describes these tests.

Road tests:

To ensure consistent testing conditions, a portion of the George Washington Parkway in the Washington, D.C.

area and its surrounding roads were used as a testing track. These roads have curves and traffic signs which exposed test subjects to both stop/go and side-to-side accelerations which commonly induce motion sickness. These roads also lacked stop lights or any irregular traffic patterns which could cause inconsistent testing. Throughout the track, the driver had certain milestones to check speed in order to, again, keep the testing as consistent as possible.

The tests were done with a plurality of test subjects (at least 10) without a control device or an experimental device. Each test subject sat in the back seat of a car and were asked to begin reading at the entrance ramp of the George Washington Parkway. A timer was started and the subjects were instructed to alert the proctors when they experienced the first symptoms of motion sickness. At that moment the timer was stopped, the windows were rolled down, and sufficient time was given for the subject to fully recover from any motion sickness experienced. Once a full recovery had occurred the test was repeated using an experimental vestibular device or a control device that looked and sounded similar to the experimental device or an over the counter acupressure device.

The tests without an experimental device or control device were always conducted first. Thereafter, to determine the order of the other tests with a control device or an experimental device a coin was flipped when the choice was between an experimental device and similar control device or a six-sided di was used to decide the order when the choice was between an experimental device, control device or acupressure device.

Table 1 below sets forth recorded times (measured in seconds) that a test subject was able to ride without experiencing symptoms of motion sickness, and weightings based on how long it took the subject to experience motion sickness compared with a reference or baseline (i.e., the individual did not wear a control device or experimental device). Several test subjects using an experimental device ended the test before they felt any symptoms (e.g., nausea) due to muscle fatigue from holding it, or because the subject decided they weren't getting sick and electing to end the test. The 'Did Nausea End the Trial' column indicates whether the subject eventually felt motion sickness while using an experimental device or if the test ended for other reasons:

TABLE I

|  | Nothing | Control | Vibe | Psiband | W control | W Vibe | W Psiband | Did Nausea End the Trial |
|---|---|---|---|---|---|---|---|---|
| Subject 1 | 220 | 256 | 558 |  | 1.16 | 2.54 |  | N |
| Subject 2 | 326 | 246 | 523 |  | 0.75 | 1.60 |  | Y |
| Subject 3 | 48 | 97 | 367 |  | 2.02 | 7.65 |  | Y |
| Subject 4 | 296 | 264 | 701 |  | 0.89 | 2.37 |  | N |
| Subject 5 | 232 | 373 | 693 |  | 1.61 | 2.99 |  | Y |
| Subject 6 | 153 | 163 | 424 |  | 1.07 | 2.77 |  | N |
| Subject 7 | 101 | 94 | 356 |  | 0.93 | 3.52 |  | Y |
| Subject 8 | 206 | 195 | 393 |  | 0.95 | 1.91 |  | N |
| Subject 9 | 103 | 112 | 441 | 107 | 1.09 | 4.28 | 1.04 | N |
| Subject 10 | 138 | 192 | 324 | 102 | 1.39 | 2.35 | 0.74 | Y |

The results from the tests indicate that the experimental devices provided relief from the symptoms of motion sickness. With only one exception, all test subjects were able to travel at least twice as long with an experimental device without experiencing the symptoms of motion sickness as compared to the tests where they did not use an experimental device or a control device (subject 8 ended the test slightly before the 2x mark due to muscle fatigue). From these experiments, and using the equation provided by the National Institutes of Health (Equation (1) below), the inventors have a confidence level of 95% that the experimental devices provide effective treatment for motion sickness.

$$Sample\ Size = \frac{Z_{1-\frac{\alpha}{2}}^2 p(1-p)}{d^2} \qquad EQ.\ (1)$$

where

$$Z_{1-\frac{\alpha}{2}}$$

is the standard normal variate (for a less than 5% error (95% confidence) 1.96 is used), p is the expected proportion of the population to suffer from the condition based on previous studies (because motion sickness is such a spectrum 50% was used to maximize the sample size required), and d is the absolute error in precision permitted in data collection (because there is over a 100% increase in how long subjects were able to travel, a 35% absolute error in precision would still indicate an experimental device was effective at delaying the symptoms of motion sickness).

Using Equation (1) the sample size applicable to the test results above were calculated as follows:

$$Sample\ Size = \frac{Z_{1-\frac{\alpha}{2}}^{2}p(1-p)}{d^2} = \frac{(1.96)^2(0.5)(1-0.5)}{(0.35)^2} = 7.84$$

With the current sample set being greater than 7.84 (i.e., 8) the test results indicate that the experimental devices have, with a greater than 95% certainty, that they provide effective relief from the symptoms of motion sickness, an, in particular, delaying the onset of symptoms associated with motion sickness brought on by reading in a car.

An additional 30 test subjects were asked to perform tasks in the backseat of a car, boat, train, and plane which typically induced motion sickness while wearing an experimental vestibular device for a minimum of 20 minutes. All but one test subject indicated that their discomfort was significantly reduced or eliminated.

Virtual reality tests:

A plurality of test subjects were exposed to two virtual reality environments which exposed them to angular and lateral motion. Initially, the test subjects were not provided with an experimental device. The time it took for the test subjects to develop discomforting symptoms was measured. Thereafter, the test subjects were provided with an experimental vestibular device, and the time until the test subjects developed discomforting symptoms was again measured. The results indicated that the test subjects provided with an experimental device could be exposed to the virtual environments without feeling discomforting symptoms for a time period that was twice as long as the time period during which the subjects were exposed to the same environments without using the experimental device (with many never feeling discomforting symptoms with the device).

A plurality of test subjects 'rode' virtual roller coasters for a set amount of time both with, and without, an experimental device. In each scenario, the test subjects were asked immediately afterwards to fill out a conventional motion sickness questionnaire as well as again 10 minutes after the test. All test subjects who experienced discomfort expressed less motion sickness initially and a faster recovery from motion sickness while using an experimental device.

Vertigo testing:

Five test subjects who suffered from vertigo were tested. Two of them suffered from vestibular migraine, one had not identified the cause, and two were caused by alcohol consumption. An experimental device provided immediate reduction in symptoms in all cases. The symptoms returned soon after the experimental device was removed and were reduced again when the device was reapplied.

Tinnitus testing:

A test subject that suffered from tinnitus wore an experimental device reported their tinnitus was relived while the device was worn. It returned shortly after the device was removed.

**Claims**

1.   A system (1) for reducing the symptoms of maladies of the vestibular system comprising:

one or more vestibular devices (2), each device (2) operable to generate one or more vibratory signals that cause a de-stimulation or masking of the vestibular system of an individual, wherein each of the one or more vestibular devices (2) comprises:

a signal generator (2c) for generating one or more initial signals;
an amplifying section (2b) for receiving the one or more initial signals and amplifying the one or more initial signals by an amount sufficient to produce one or more amplified signals that de-stimulate the vestibular system; and
a bone conduction transducer (2a) operable to receive the one or more amplified signals and generate the one or more vibratory signals, the bone conduction transducer (2a) operable to generate the one or more vibratory signals having a majority of power contained in a fundamental frequency of the one or more vibratory signals, the one or more vibratory signals including signals at one or more harmonics of the fundamental frequency in the range 10 to 300 Hz,

the one or more vestibular devices (2) configured to be positioned on a head of the individual such that the one or more vibratory signals are allowed to propagate through to the vestibular system over bone.

2. The system as in claim 1, wherein the bone conduction transducer comprises:
a coil operable to generate a magnetic field in response to receiving the one or more amplified signals.

3. The system as in claim 2, wherein the bone conduction transducer includes:

a magnet configured to oscillate along an axis;
a rod configured to enable motion of the magnet along a direction parallel to the axis and to restrict motion of the magnet in a direction not parallel to the axis; and
bucking magnets configured to apply opposing forces upon the magnet such that the magnet oscillates about an equilibrium position.

4. The system as in claim 1, further comprising a band, wherein the band is configured to position the one or more vestibular devices at positions that enable the one or more vibratory signals to propagate through bone to the vestibular system.

5. The system as in claim 1, further comprising at least one of a headband, an audio headphone, an earcup, a car seat, a pillow, a virtual reality device, glasses, a hat, a hair accessory, a helmet, or a headset,
wherein the one or more vestibular devices are integrated into, or connected to, the at least one of: the headband, the audio headphone, the earcup, the car seat, the pillow, the virtual reality device, the glasses, the hat, the hair accessory, the helmet, or the headset.

6. The system as in claim 1, further comprising:

a sensor, wherein the one or more vestibular devices are integrated into, or connected to, the sensor, the sensor configured to measure one or more factors including at least one of temperatures, noise levels, vibrations, accelerations, de-accelerations, velocities, pressures, or moisture; and
a processor configured to receive information from the sensor indicative of the one or more factors and control operation of at least one of the signal generator, the amplifier, or the bone conduction transducer of each of the one or more vestibular devices based on the information.

7. The system as in claim 1, where the system is configured to reduce symptoms of at least one of motion sickness, dizziness, virtual reality sickness, vertigo or tinnitus.

8. The system as in claim 1, wherein the bone conduction transducer includes:

a magnet configured to oscillate about an equilibrium position; and
a spring secured to the magnet, and the one or more vibratory signals are resonant frequencies of the spring and the magnet.

9. The system as in claim 1, wherein the bone conduction transducer includes a padded material such that, when the bone conduction transducer comes into contact with a skin or body of the individual, the contact is spread over a larger area to reduce pressure.

**10.** The system as in claim 1, wherein the bone conduction transducer includes a friction reducing material such that, when the bone conduction transducer comes into contact with a kin or body of the individual, the contact is less abrasive.

**11.** The system as in claim 1, wherein the bone conduction transducer includes:

a magnet configured to oscillate about an equilibrium position;
one or more endcaps; and
one or more springs secured to the one or more endcaps and the magnet, the one or more springs configured to apply forces upon the magnet to control the oscillation of the magnet about the equilibrium position.

**12.** The system as in claim 1, wherein the bone conduction transducer is configured to deliver the one or more vibratory signals to the portion of the head of the individual such that the one of more vibratory signals propagate to the otoliths, endolymph, and hair follicles of the vestibular system.

**Patentansprüche**

**1.** System (1) zur Verringerung der Symptome von Leiden des vestibulären Systems, umfassend:

eine oder mehrere vestibuläre Vorrichtungen (2), wobei jede Vorrichtung (2) funktionell ist, um ein oder mehrere Vibrationssignale zu generieren, die eine Destimulation oder Maskierung des vestibulären Systems eines Individuums bewirken, wobei jede der einen oder mehreren vestibulären Vorrichtungen (2) umfasst:

einen Signalgenerator (2c) zum Generieren von einem oder mehreren Anfangssignalen;
einen Verstärkungsabschnitt (2b) zum Empfangen des einen oder der mehreren Anfangssignale und Verstärken des einen oder der mehreren Anfangssignale um einen Betrag, der ausreicht, um ein oder mehrere verstärkte Signale zu produzieren, die das vestibuläre System destimulieren; und
einen Knochenleitungswandler (2a), der funktionell ist, um das eine oder die mehreren verstärkten Signale zu empfangen und das eine oder die mehreren Vibrationssignale zu generieren, wobei der Knochenleitungswandler (2a) funktionell ist, um das eine oder die mehreren Vibrationssignale zu generieren, wobei ein überwiegender Teil der Leistung in einer Grundfrequenz des einen oder der mehreren Vibrationssignale enthalten ist, wobei das eine oder die mehreren Vibrationssignale Signale mit einer oder mehreren Oberschwingungen der Grundfrequenz im Bereich von 10 bis 300 Hz einschließen,

wobei die eine oder mehreren vestibulären Vorrichtungen (2) ausgestaltet ist/sind, um auf einem Kopf des Individuums positioniert zu werden, so dass das eine oder die mehreren Vibrationssignale sich über Knochen bis zu dem vestibulären System ausbreiten können.

**2.** Vorrichtung nach Anspruch 1, wobei der Knochenleitungswandler umfasst:
eine Spule, die funktionell ist, um in Reaktion auf das Empfangen des einen oder der mehreren verstärkten Signale ein Magnetfeld zu generieren.

**3.** System nach Anspruch 2, wobei der Knochenleitungswandler einschließt:

einen Magneten, der ausgestaltet ist, um entlang einer Achse zu oszillieren;
einen Stab, der ausgestaltet ist, um Bewegung des Magneten entlang einer Richtung parallel zu der Achse zu ermöglichen und Bewegung des Magneten in einer Richtung, die nicht parallel zu der Achse ist, zu beschränken; und
Kompensationsmagnete, die ausgestaltet sind, um Gegenkräfte auf den Magneten auszuüben, so dass der Magnet um eine Gleichgewichtsposition oszilliert.

**4.** System nach Anspruch 1, des Weiteren umfassend ein Band, wobei das Band ausgestaltet ist, um die eine oder mehreren vestibulären Vorrichtungen in Positionen zu positionieren, die ermöglichen, dass sich das eine oder die mehreren Vibrationssignale durch Knochen bis zu dem vestibulären System ausbreitet/ausbreiten.

**5.** System nach Anspruch 1, des Weiteren umfassend mindestens eines von: einem Stirnband, einem Audiokopfhörer, einem Ohrpolster, einem Autositz, einem Kissen, einer Virtual Reality-Vorrichtung, Brillengläsern, einem Hut, einem Haarschmuck, einem Helm oder einem Headset,
wobei die eine oder mehreren vestibulären Vorrichtungen in mindestens eines von: dem Stirnband, dem Audiokopfhörer, dem Ohrpolster, dem Autositz, dem Kissen, der Virtual Reality-Vorrichtung, den Brillengläsern, dem Hut, dem Haarschmuck, dem Helm oder dem Headset integriert sind oder damit verbunden sind.

6. System nach Anspruch 1, des Weiteren umfassend:
einen Sensor, wobei die eine oder mehreren vestibulären Vorrichtungen in den Sensor integriert oder damit verbunden ist/sind, wobei der Sensor ausgestaltet ist, um einen oder mehrere Faktoren zu messen, einschließlich mindestens eines von:

Temperaturen, Rauschpegeln, Vibrationen, Beschleunigungen, Verzögerungen, Geschwindigkeiten, Drücken oder Feuchtigkeit; und
einen Prozessor, der ausgestaltet ist, um Informationen von dem Sensor zu empfangen, welche den einen oder die mehreren Faktoren angeben, und den Betrieb von mindestens einem von dem Signalgenerator, dem Verstärker oder dem Knochenleitungswandler von jeder der einen oder den mehreren vestibulären Vorrichtung basierend auf den Informationen zu steuern.

7. System nach Anspruch 1, wobei das System ausgestaltet ist, um Symptome von mindestens einem von: Kinetose, Schwindel, Virtual Reality-Übelkeit, Vertigo oder Tinnitus zu verringern.

8. System nach Anspruch 1, wobei der Knochenleitungswandler einschließt:

einen Magneten, der ausgestaltet ist, um um eine Gleichgewichtsposition zu oszillieren; und
eine Feder, die an dem Magneten gesichert ist, und wobei das eine oder die mehreren Vibrationssignale Resonanzfrequenzen der Feder und des Magneten ist/sind.

9. System nach Anspruch 1, wobei der Knochenleitungswandler ein gepolstertes Material einschließt, so dass, wenn der Knochenleitungswandler in Kontakt mit einer Haut oder einem Körper des Individuums kommt, der Kontakt über eine größere Fläche verteilt wird, um den Druck zu verringern.

10. System nach Anspruch 1, wobei der Knochenleitungswandler ein reibungsverminderndes Material einschließt, so dass, wenn der Knochenleitungswandler in Kontakt mit einer Haut oder einem Körper des Individuums kommt, der Kontakt weniger abrasiv ist.

11. System nach Anspruch 1, wobei der Knochenleitungswandler einschließt:

einen Magneten, der ausgestaltet ist, um um eine Gleichgewichtsposition zu oszillieren;
ein oder mehrere Abschlussstücke; und
eine oder mehrere Federn, die an dem einen oder den mehreren Abschlussstücken und dem Magneten gesichert ist/sind, wobei die eine oder mehreren Federn ausgestaltet ist/sind, um Kräfte auf den Magneten auszuüben, um die Oszillation des Magneten um die Gleichgewichtsposition zu steuern.

12. System nach Anspruch 1, wobei der Knochenleitungswandler ausgestaltet ist, um das eine oder die mehreren Vibrationssignale an den Anteil des Kopfes des Individuums abzugeben, so dass das eine oder die mehreren Vibrationssignale zu den Otolithen, der Endolymphe und den Haarfollikeln des vestibulären Systems ausgebreitet wird/werden.

**Revendications**

1. Système (1) permettant de réduire les symptômes de maladies du système vestibulaire comprenant :

un ou plusieurs dispositifs vestibulaires (2), chaque dispositif (2) étant utilisable pour générer un ou plusieurs signaux vibratoires qui provoquent une déstimulation ou un masquage du système vestibulaire d'un individu, dans lequel chacun des un ou plusieurs dispositifs vestibulaires (2) comprend :

un générateur de signaux (2c) pour générer un ou plusieurs signaux initiaux ;
une section d'amplification (2b) pour recevoir les un ou plusieurs signaux initiaux et amplifier les un ou plusieurs signaux initiaux d'une quantité suffisante pour produire un ou plusieurs signaux amplifiés qui déstimulent le système vestibulaire ; et

un transducteur à conduction osseuse (2a) utilisable pour recevoir les un ou plusieurs signaux amplifiés et générer les un ou plusieurs signaux vibratoires, le transducteur à conduction osseuse (2a) étant utilisable pour générer les un ou plusieurs signaux vibratoires dont la majeure partie de la puissance est contenue dans une fréquence fondamentale des un ou plusieurs signaux vibratoires, les un ou plusieurs signaux vibratoires comprenant des signaux à une ou plusieurs harmoniques de la fréquence fondamentale dans la plage de 10 à 300 Hz,

les un ou plusieurs dispositifs vestibulaires (2) configurés pour être positionnés sur la tête de l'individu de telle sorte que les un ou plusieurs signaux vibratoires puissent se propager à travers le système vestibulaire sur l'os.

2. Système selon la revendication 1, dans lequel le transducteur à conduction osseuse comprend :
une bobine utilisable pour générer un champ magnétique en réponse à la réception des un ou plusieurs signaux amplifiés.

3. Système selon la revendication 2, dans lequel le transducteur à conduction osseuse comporte:

un aimant configuré pour osciller le long d'un axe ;
une tige configurée pour permettre un mouvement de l'aimant le long d'une direction parallèle à l'axe et pour restreindre le mouvement de l'aimant dans une direction non parallèle à l'axe ; et
des aimants de compensation configurés pour appliquer des forces opposées sur l'aimant de telle sorte que l'aimant oscille autour d'une position d'équilibre.

4. Système selon la revendication 1, comprenant en outre une bande, dans lequel la bande est configurée pour positionner les un ou plusieurs dispositifs vestibulaires à des positions qui permettent que les un ou plusieurs signaux vibratoires se propagent à travers l'os jusqu'au système vestibulaire.

5. Système selon la revendication 1, comprenant en outre au moins l'un des éléments suivants : un bandeau, un casque audio, une oreillette, un siège de voiture, un oreiller, un dispositif de réalité virtuelle, des lunettes, un chapeau, un accessoire pour cheveux, un casque ou un casque d'écoute,
dans lequel les un ou plusieurs dispositifs vestibulaires sont intégrés ou connectés à au moins un des éléments suivants : le bandeau, le casque audio, l'oreillette, le siège de voiture, l'oreiller, le dispositif de réalité virtuelle, les lunettes, le chapeau, l'accessoire pour cheveux, le casque ou le casque d'écoute.

6. Système selon la revendication 1, comprenant en outre :

un capteur, dans lequel les un ou plusieurs dispositifs vestibulaires sont intégrés ou connectés au capteur, le capteur étant configuré pour mesurer un ou plusieurs facteurs comprenant au moins l'un parmi : des températures, des niveaux de bruit, des vibrations, des accélérations, des décélérations, des vitesses, des pressions ou de l'humidité ; et
un processeur configuré pour recevoir du capteur des informations faisant état des un ou plusieurs facteurs et pour commander le fonctionnement d'au moins l'un parmi le générateur de signaux, l'amplificateur ou le transducteur à conduction osseuse de chacun des un ou plusieurs dispositifs vestibulaires sur la base des informations.

7. Système selon la revendication 1, le système étant configuré pour réduire des symptômes d'au moins un parmi : le mal des transports, le vertige, le mal de la réalité virtuelle, le vertige ou l'acouphène.

8. Système selon la revendication 1, dans lequel le transducteur à conduction osseuse comprend :

un aimant configuré pour osciller autour d'une position d'équilibre ; et
un ressort fixé à l'aimant, et les un ou plusieurs signaux vibratoires sont des fréquences de résonance du ressort et de l'aimant.

9. Système selon la revendication 1, dans lequel le transducteur à conduction osseuse comprend un matériau rembourré de telle sorte que, lorsque le transducteur à conduction osseuse entre en contact avec la peau ou le corps de l'individu, le contact soit réparti sur une plus grande surface pour réduire la pression.

**10.** Système selon la revendication 1, dans lequel le transducteur à conduction osseuse comprend un matériau réduisant la friction de telle sorte que, lorsque le transducteur à conduction osseuse entre en contact avec la peau ou le corps de l'individu, le contact soit moins abrasif.

**11.** Système selon la revendication 1, dans lequel le transducteur à conduction osseuse comprend :

un aimant configuré pour osciller autour d'une position d'équilibre ;
un ou plusieurs embouts ; et
un ou plusieurs ressorts fixés aux un ou plusieurs embouts et à l'aimant, les un ou plusieurs ressorts étant configurés pour appliquer des forces sur l'aimant afin de commander l'oscillation de l'aimant autour de la position d'équilibre.

**12.** Système selon la revendication 1, dans lequel le transducteur à conduction osseuse est configuré pour délivrer les un ou plusieurs signaux vibratoires à la partie de la tête de l'individu de telle sorte que les un ou plusieurs signaux vibratoires se propagent aux otolithes, à l'endolymphe et aux follicules pileux du système vestibulaire.

*FIG. 1*

FIG. 2

FIG. 3

FIG. 4

FIG. 5

500

501

502

FIG. 6

500

503

502

501

FIG. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20160089298 A

- US 20140309718 A

**Non-patent literature cited in the description**

- **H. SOHMER et al.** Effect of noise on the vestibular system - Vestibular evoked potential studies in rats. *Noise Health,* 1999, vol. 2, 41